# EUROPEAN PATENT SPECIFICATION

(11) **EP 4 003 399 B1**
(45) Date of publication and mention of the grant of the patent: **04.11.2026**
(21) Application number: 20847042.7
(22) Date of filing: 22.07.2020
(51) Int. Cl.: A61K 38/17, C07K 16/28, A61P 21/00, A61K 47/68, C07K 14/71

(54) **TGFBETARII-FC FOR USE IN THE PREVENTION OR TREATMENT OF FATTY DEGENERATION OF SKELETAL MUSCLE**
TGFBETARII-FC ZUR VERWENDUNG BEI DER PRÄVENTION ODER BEHANDLUNG VON FETTIGER DEGENERATION DER SKELETTMUSKULATUR
TGFBETARII-FC POUR UNE UTILISATION DANS LA PRÉVENTION OU LE TRAITEMENT DE LA DÉGÉNÉRESCENCE GRAISSEUSE DU MUSCLE SQUELETTIQUE

(30) Priority: 26.07.2019 US 201962879009 P
(43) Date of publication of application: 01.06.2022
(73) Proprietor: Board Of Trustees Of Michigan State University, East Lansing, Michigan 48824-1046 (US)
(72) Inventor: MARTINEZ-HACKERT, Erik, Okemos, Michigan 48864 (US); FLOER, Monique, Okemos, Michigan 48864 (US)
(74) Representative: August Debouzy
(86) International application number: PCT/US2020/043119
(87) International publication number: WO 2021/021528

(56) References cited:
- WO-A1-98/48024
- KR-A- 20180 035 884
- US-A1- 2016 287 664
- ACCORNERO FEDERICA ET AL: "Myofiber-specific inhibition of TGF[beta] signaling protects skeletal muscle from injury and dystrophic disease in mice", vol. 23, no. 25, 20 December 2014 (2014-12-20), GB, pages 6903 - 6915, XP093032304, ISSN: 0964-6906, Retrieved from the Internet <URL:https://www.ncbi.nlm.nih.gov/pmc/articles/PMC4271062/pdf/ddu413.pdf> DOI: 10.1093/hmg/ddu413
- SULSAL HAQUE ET AL: "Transforming growth factor-?: A therapeutic target for cancer", HUMAN VACCINES & IMMUNOTHERAPEUTICS, vol. 13, no. 8, 2 June 2017 (2017-06-02), US, pages 1741 - 1750, XP055629801, ISSN: 2164-5515, DOI: 10.1080/21645515.2017.1327107
- STEPIEN DAVID M. ET AL: "Tuning Macrophage Phenotype to Mitigate Skeletal Muscle Fibrosis", vol. 204, no. 8, 15 April 2020 (2020-04-15), US, pages 2203 - 2215, XP093032296, ISSN: 0022-1767, Retrieved from the Internet <URL:https://journals.aai.org/jimmunol/article-pdf/204/8/2203/1458007/ji1900814.pdf> DOI: 10.4049/jimmunol.1900814
- KAJI DEEPAK A ET AL: "Tgf? signaling is required for tenocyte recruitment and functional neonatal tendon regeneration", vol. 9, 5 June 2020 (2020-06-05), XP055797033, Retrieved from the Internet <URL:https://www.ncbi.nlm.nih.gov/pmc/articles/PMC7324157/pdf/elife-51779.pdf> DOI: 10.7554/eLife.51779
- DAVIES, MICHAEL R., LIU XUHUI, LEE LAWRENCE, LARON DOMINIQUE, NING ANNE Y., KIM HUBERT T., FEELEY BRIAN T.: "TGF-p Small Molecule Inhibitor SB431542 Reduces Rotator Cuff Muscle Fibrosis and Fatty Infiltration By Promoting Fibro/Adipogenic Progenitor Apoptosis", PLOS ONE, vol. 11, no. 5, 17 May 2016 (2016-05-17), pages 1 - 15, XP055791345, DOI: 10.1371/journal.pone.0155486
- PAGANO, AF ET AL.: "Muscle Resting and TGF-beta Inhibitor Treatment Prevent Fatty Infiltration Following Skeletal Muscle Injury", CELLULAR PHYSIOLOGY AND BIOCHEMISTRY, vol. 53, 12 June 2019 (2019-06-12), pages 62 - 75, XP055791347, DOI: 10.33594/000000121
- MURAOKA, RS ET AL.: "Blockade of TGF-beta inhibits mammary tumor cell viability, migration, and metastases", THE JOURNAL OF CLINICAL INVESTIGATION, vol. 109, no. 12, June 2002 (2002-06-01), pages 1551 - 1559, XP055547017, DOI: 10.1172/JCI200215234
- LOISELLE, ALAYNA E ET AL.: "Development of Antisense Oligonucleotide (ASO) Technology Against Tgf-P Signaling to Prevent Scarring During Flexor Tendon Repair", JOURNAL OF ORTHOPAEDIC RESEARCH, vol. 33, no. 6, June 2015 (2015-06-01), pages 859 - 866, XP055791369, DOI: 10.1002/jor.22890
- LEMOS, DARIO R, BABAEIJANDAGHI FARSHAD, LOW MARCELA, CHANG CHIH-KAI, LEE SUNNY T, FIORE DANIELA, ZHANG REGAN-HENG, NATARAJAN ANURA: "Nilotinib reduces muscle fibrosis in chronic muscle injury by promoting TNF-mediated apoptosis of fibro/adipogenic progenitors", NATURE MEDICINE, vol. 21, no. 7, July 2015 (2015-07-01), pages 786 - 794, XP055791374, DOI: 10.1038/nm.3869

## Description

### CROSS-REFERENCE TO RELATED APPLICATIONS

This application claims the benefit under 35 U.S.C. §119(e) of U.S. Provisional Patent Application No. 62/879,009 filed on July 26, 2019.

### FIELD

The present invention generally relates to TGFβRII-Fc as a selective TGF-β inhibitor, for use in the prevention or treatment of fatty degeneration of skeletal muscle.

### BACKGROUND

The background description includes information that may be useful in understanding the present disclosure. It is not an admission that any of the information provided herein is prior art or relevant to the presently claimed invention, or that any publication specifically or implicitly referenced is prior art.

The transforming growth factor beta (TGF-β) family is a well-known family of structurally related proteins that control proliferation, differentiation, development regulation and other functions in many cell types. The TGF-β family includes canonical TGF-β, which forms a group of three isoforms, TGF-β1, TGF-β2, and TGF-β3. The three isoforms, TGF-β1-3, have long been known to have distinct functions. They are differentially expressed in various tissues and at distinct times during development.

Fatty degeneration ("FD") of muscle tissue can occur following injuries, repair surgeries, such as rotator cuff ("RC") repair surgery, and other conditions. Various approaches have been taken to attempt to inhibit TGF-β signaling, such as monoclonal antibodies and small molecules. Notably, targeting TGF-β pathways with a small molecule inhibitor (SB431542) in a mouse model of massive RC tears was reported to reduce FD of relevant muscles. However, SB431542 will not work clinically due to its broad specificity and poor pharmacological properties.

Additionally, fusion proteins comprising the TGF-β type II receptor linked to a portion of an immunoglobulin constant region ("TGFβRII-Fc") are reported in published International Patent Application, WO 1998/048024 and U.S. Patent No. 9,809,637.

Thus, even though various selective TGF-β inhibitors are known in the art, there are currently no treatments that prevent or mitigate FD of muscles in humans. Therefore, a need exists to provide improved compositions and methods to prevent and/or treat FD.

### SUMMARY

The invention relates to TGFβRII-Fc for use in the prevention or treatment of fatty degeneration of skeletal muscle in a subject in need thereof.

The invention is set out in the appended set of claims. The references to the methods of treatment by therapy throughout this description are to be interpreted as references to TGFβRII-Fc for use in the prevention or treatment of fatty degeneration of skeletal muscle.

In further embodiments not claimed, a method of inhibiting differentiation of fibro-adipogenic progenitor cells (FAPs) into adipocytes is also described herein. The method comprises contacting the intercellular compartment surrounding a FAP cell with an effective amount of a selective TGF-β inhibitor.

In the invention, the selective TGF-β inhibitor is a TGFβRII-Fc as described herein.

In further particular embodiments, the selective TGF-β inhibitor is a TGFβRII-Fc from an animal species that is used to prevent, reverse, reduce the occurrence of and/or treat FD of skeletal muscle in the same animal species.

Various objects, features, aspects, and advantages will become more apparent from the following detailed description of preferred embodiments, along with the accompanying drawing in which like numerals represent like components.

### BRIEF DESCRIPTION OF THE DRAWINGS

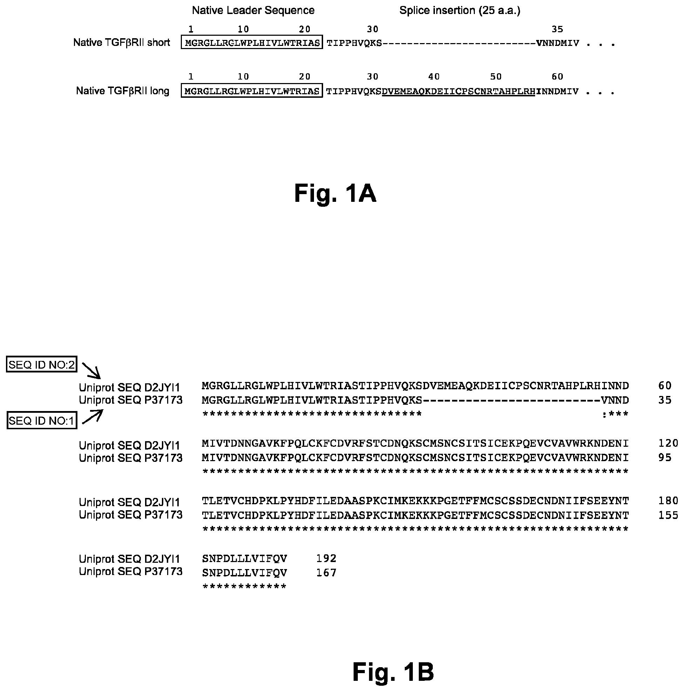

Figs. 1A-1B show the two splice forms of TGFβRII that occur in nature, the native short-form and the native long-form. Fig. 1A shows that the long-form has an insertion of 25 amino acids after amino acid 31, and the amino acid following this insertion is changed from Val to Ile (shown in bold). Fig. 1B shows the sequences of the extracellular domains (ECDs) of both forms of TGFβRII. Uniprot SEQ P37173 (SEQ ID NO:1 herein ) is the short-form; Uniprot SEQ D2JYI1 (SEQ ID NO:2 herein) is the long-form.

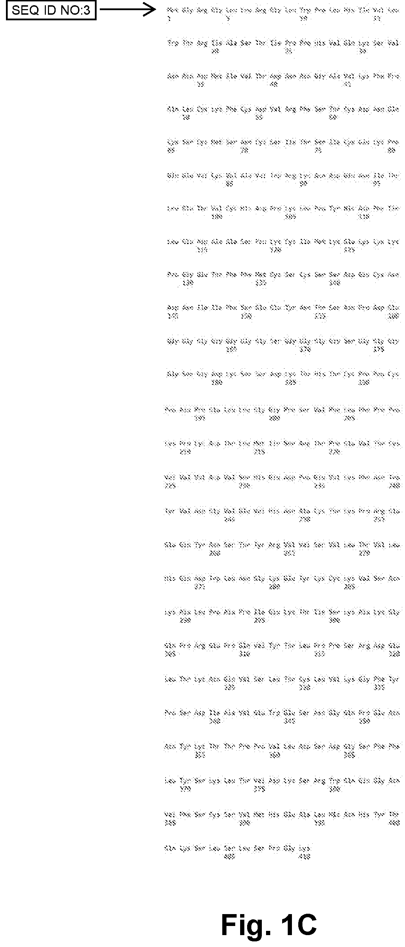

Fig. 1C is the amino acid sequence of a TGFβRII-Fc (SEQ ID NO:3) used in Examples 1-3 herein.

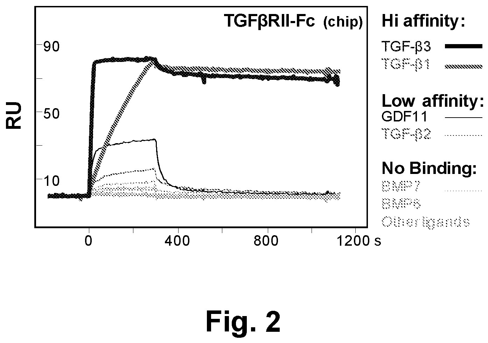

Fig. 2 shows the binding specificity of a TGFβRII-Fc. The figure is an image of an SPR-sensogram that shows binding of TGFβRII-Fc (SEQ ID NO:3) to TGF-β1 and TGF-β3, but not TGF-β2. See Aykul, S. and Martinez-Hackert, E. Transforming Growth Factor- β family ligands can function as antagonists by competing for type II receptor binding. Journal of Biological Chemistry 291: 10792-10804 (2016).

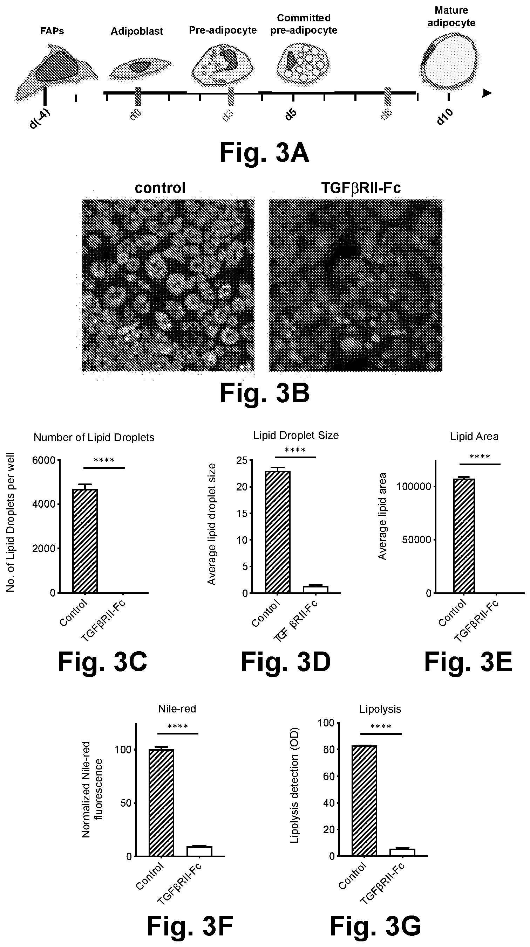

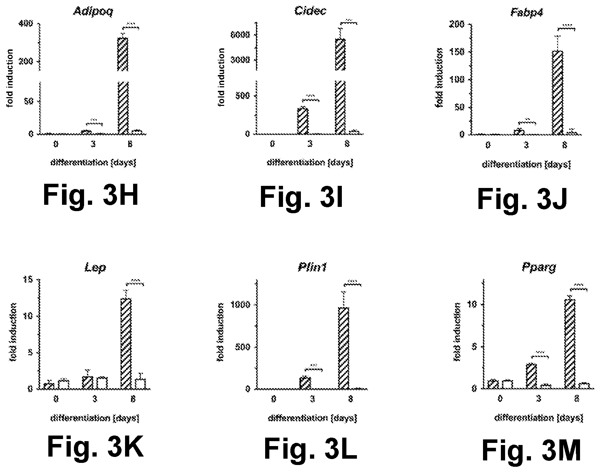

Figs. 3A-3M show inhibition of differentiation of fibro-adipogenic 3T3-L1 progenitor cells into adipocytes by a TGFβRII-Fc. Fig. 3A is a schematic showing how fibro-adipogenic progenitor cells (FAPs) differentiate into adipocytes. See Aykul, S., Maust, J., Floer, M. and Martinez-Hackert, M. TGF-B Family Inhibitors Blunt Adipogenesis Via Non-Canonical Regulation Of SMAD Pathways. bioRxiv. Posted online on March 14, 2020. Figs. 3B-3G shows immunofluorescence analysis of 3T3-L1 cells that were grown in adipocyte differentiation medium for 8 days, with vehicle control (PBS) or 300 nM TGFβRII-Fc. Fig. 3B shows an image of cells that were stained for lipids with Nile-red (green); nuclei were stained with DAPI (magenta). Fig. 3C shows the quantification of the number of liqid droplets, Fig. 3D shows the average size of liqid droplets, Fig. 3E shows the average lipid area, Fig. 3F shows the normalized Nile-red fluorescence and Fig. 3G shows lipolysis activity. Hatched bars show vehicle control and white bars show TGFβRII-Fc treated cells. Figs. 3H-3M show gene expression analysis of 3T3-L1 cells grown for different lengths of time in differentiation medium. Induction of adipocyte marker gene expression was analyzed by qRT-PCR on day 0, day 3 and day 8 in vehicle control (hatched bars) and TGFβRII-Fc treated cells (white bars). Data is shown, as fold induction after normalization to *Rpl4,* for *Adipoq* in Fig. 3H, for *Cidec* in Fig. 3I, for *Fabp4* in Fig. 3J, for *Lep* in Fig. 3K, for *Plin1* in Fig. 3L, and for *Pparg* in Fig. 3M. Significance was determined by two-way ANOVA and Sidaki's or Dunnett's multiple comparisons tests. (* p<0.05; **p<0.01; ***p<0.001; ****p<0.0001).

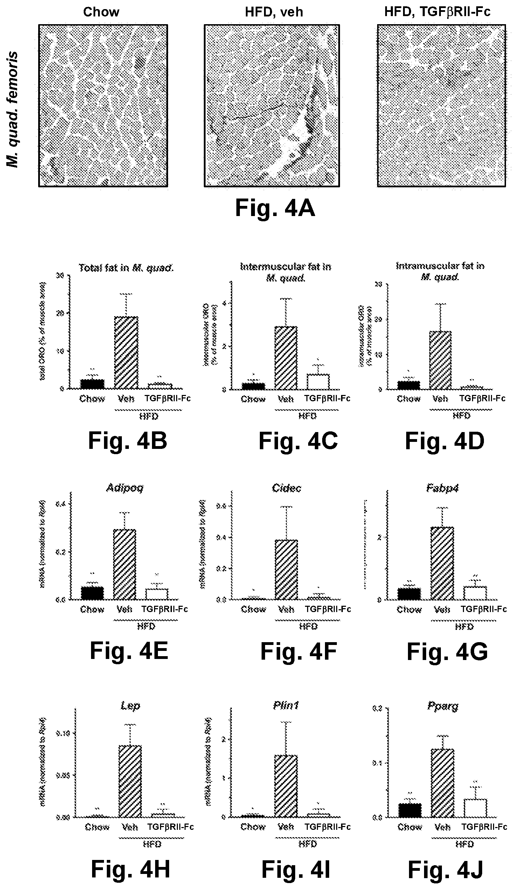

Figs. 4A-4J show inhibition of fatty infiltration ("FI") in the *Musculus quadriceps femoris* of DIO mice by a TGFβRII-Fc. Fig. 4A shows pictures of muscles that were sectioned and stained with hematoxylin (blue) followed by Oil Red O (ORO). ORO detects intramuscular fat due to lipid droplets that form inside muscle fibers as well as intermuscular fat, which is a result of adipocytes growing in between muscle fibers (FI). Fig. 4B shows the quantification of total fat, Fig. 4C shows quantification of intermuscular fat, and Fig. 4D shows quantification of intramuscular fat from the quadriceps of mice (n=3). Fig. 4E-4J show gene expression analysis of quadriceps muscles. Gene expression was analyzed by qRT-PCR and normalized to the *Rpl4* household gene. Data is shown as fold expression over chow fed mice for the fat cell marker genes *Adipoq* in Fig. 4E, for *Cidec* in Fig. 4F, for *Fabp4* in Fig. 4G, for *Lep* in Fig. 4H, for *Plin1* in Fig. 4I, and for *Pparg* in Fig. 4J. Significance was determined by one-way ANOVA followed by post-hoc Tukey's tests. (*p<0.05; **p<0.01)

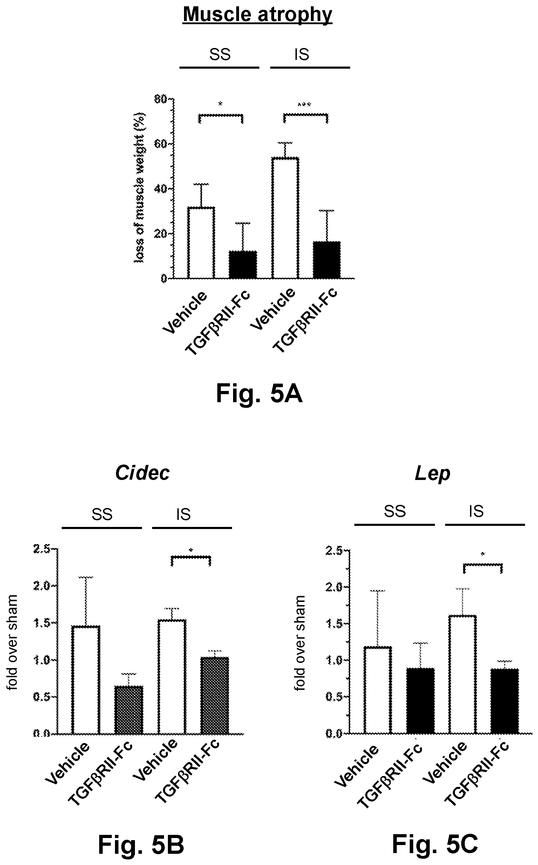

Figs. 5A-5C show inhibition of muscle atrophy and FI in shoulder muscles after induced RC injury by a TGFβRII-Fc. Fig. 5A shows quantification of the loss in weight wet of supraspinatus (SS) and infraspinatus (IS) muscles from appropriate animals (n=6). The muscles from the injury side were compared to the muscles in the sham operated side of the same mouse. Data are represented as % loss of muscle weight after induced RC injury by comparing the injured to the sham operated muscles (* p<0.05; ***p<0.001). Fig. 5B and C show gene expression analysis of shoulder muscles (n=4). Gene expression was analyzed by qRT-PCR and normalized to the *Rpl4* household gene. Data is shown as gene expression in injured compared to sham operated muscles for *Cidec* in Fig. 5B and for *Lep* in Fig. 5C. Significance was determined by one-way ANOVA followed by post-hoc Tukey's tests. (*p<0.05).

### DETAILED DESCRIPTION

### I. Definitions

The following definitions refer to the various terms used above and throughout the disclosure.

The term "selective TGF-β inhibitor or antagonist" as used herein refers to a polypeptide that inhibits TGF-β1 and/or TGF-β3 signaling, but does not substantially inhibit TGF-β2 signaling.

There are two splice-forms of TGFβRII, the native TGFβRII short-form (Uniprot SEQ ID P37173 which corresponds to SEQ ID NO:1 herein), and the native TGFβRII long-form (Uniprot SEQ ID D2JYI1 which corresponds to SEQ ID NO:2 herein). The long form is a splice variant with a 25 amino acid insertion after amino acid 31, where the amino acid flanking the insertion is changed from Val to Ile (Fig. 1).

As used herein a "TGFβRII ECD polypeptide fusion" comprises a polypeptide of the extracellular domain ("ECD") of TGFβRII and a heterologous sequence. The TGFβRII portion comprises a polypeptide sequence having at least 80%, or at least 85%, or at least 90%, or at least 95% sequence identity to the amino acid sequence set forth as amino acids 23 through 166 of SEQ ID NO:1; or at least 80%, or at least 85%, at least 90%, or at least 95% sequence identity to the amino acid sequence set forth as amino acids 23 through 191 of SEQ ID NO:2, and wherein the polypeptide is capable of binding TGF-β1 and/or TGF-β3, but does not substantially bind to TGF-β2.

TGFβRII ECD polypeptide fusions include a polypeptide of TGFβRII, wherein TGFβRII comprises a polypeptide sequence beginning at any of positions 23 to 51 of SEQ ID NO:1 and ending at any of positions 143 to 166 of SEQ ID NO:1, or a sequence beginning at any of positions 23 to 76 of SEQ ID NO:2 and ending at any of positions 169 to 191 of SEQ ID NO:2, and wherein the polypeptide is capable of binding TGF-β1 and/or TGF-β3.

The heterologous sequence portion of a TGFβRII ECD polypeptide fusion can be a constant domain ("Fc") of a human IgG or albumin. When the TGFβRII portion is fused to the Fc of a human immunoglobulin, IgG, the resulting fusion protein is known as a "TGFβRII-Fc". In other words, a TGFβRII-Fc comprises or consists of the extracellular domain of the TGFβRII receptor, as described above, fused to the Fc of a human IgG. The human IgG moiety in a TGFβRII-Fc can consist of the Fc domain of human IgG1, IgG2, or IgG4. The TGFβRII portion can be fused with the Fc domain at the N-terminus or the C-terminus with a connecting linker of varying length and amino acid sequence, but preferably without secondary structure. A TGFβRII-Fc can comprise either the TGFβRII short-form or the long-form, and fusions containing either splice-form have similar binding specificities for TGF-β1 and/or TGF-β3; and they do not substantially bind TGF-β2.

TGFβRII-Fc are known fusion proteins. For example, WO 1998/048024 reports a TGFβRII-Fc and methods of making a TGFβRII-Fc where the short form of TGFβRII is fused to the Fc of human IgG1. Additionally U.S. 9,809,637 reports a TGFβRII-Fc using the long form of TGFβRII. TGFβRII-Fc is an inhibitor of TGF-β1 and/or TGF-β3. However, it does not substantially bind to/inhibit TGF-β2. See Fig. 2 which is taken from Aykul, S. and Martinez-Hackert, E. Transforming Growth Factor-β family ligands can function as antagonists by competing for type II receptor binding. Journal of Biological Chemistry 291: 10792-10804 (2016), cited above. Without being bound by theory, it is a so-called "ligand trap," which works by trapping the ligands TGF-β1 and/or TGF-β3 in the intercellular compartment. This prevents the ligands from interacting with the endogenous TGFβRII receptor and, therefore, inhibits downstream signaling. Thus, a TGFβRII-Fc can be used as selective antagonists of TGF-β1 and/or TGF-β3, but substantially not TGF-β2.

In particular embodiments, TGFβRII-Fc has at least 80%, at least 85%, at least 90%, or at least 95% similarity to the amino acid sequence set forth as SEQ ID NO:3. See Fig. 1C. In further particular embodiments, the TGFβRII-Fc comprises or consists of the amino acid sequence set forth as SEQ ID NO:3.

The term "treat" and "treatment" refers to a method for reducing, inhibiting, or otherwise ameliorating FD of skeletal muscle by administering a therapeutically effective amount of TGFβRII-Fc.

The term "fatty degeneration" ("FD") refers to the abnormal formation of fat tissue in between muscle fibers and the accompanying atrophy of muscles, which can be a result of injury or a disease/disorder.

The term "fatty infiltration" ("FI") refers to the abnormal formation of fat tissue only.

The term "administering" refers to both direct and indirect administration of a pharmaceutical composition or drug, wherein direct administration of the pharmaceutical composition or drug is typically performed by a health care professional (*e.g*., physician, nurse, etc.), and wherein indirect administration includes a step of providing or making available the pharmaceutical composition or drug to the health care professional for direct administration (*e.g*., via injection, infusion, oral delivery, topical delivery, etc.).

The term "effective amount" or "therapeutically effective amount" refers to the amount and/or dosage, and/or dosage regime necessary to bring about the desired result i.e. an amount sufficient to prevent FD in skeletal muscle in a subject, an amount sufficient to reduce the occurrence of FD in skeletal muscle in a subject, and/or an amount sufficient to treat FD in skeletal muscle in a subject by administration of TGFβRII-Fc.

The terms "subject", "individual", and "patient" interchangeably refer to a mammal, preferably a human or a non-human primate, but also domesticated mammals (*e.g*., canine or feline), laboratory mammals (*e.g*., mouse, rat, rabbit, hamster, guinea pig) and agricultural mammals (*e.g*., equine, bovine, porcine, ovine). In various embodiments, the subject can be a human (*e.g*., adult male, adult female, adolescent male, adolescent female, male child, female child) under the care of a physician or other health worker in a hospital, psychiatric care facility, as an outpatient, or other clinical context. In certain embodiments the subject may not be under the care or prescription of a physician or other health worker.

The term "surgery" or "surgical procedure" refers collectively to all therapeutic and diagnostic procedures, including procedures requiring an incision made on a subject as well as endoscopic procedures.

### II. Pharmaceutical Compositions

In some embodiments, pharmaceutical compositions are provided comprising a selective TGF-β inhibitor, such as a TGFβRII-Fc, and a pharmaceutically-acceptable carrier. For example, a TGFβRII-Fc may be formulated with a pharmaceutically-acceptable carrier.

The pharmaceutical compositions for use according to claim 1 may be formulated according to conventional pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

### III. Methods of Use

It has been discovered that TGFβRII-Fc, may be used to inhibit fibro-adipogenic progenitor cell (FAP) differentiation into adipocytes. The method, which is not claimed, comprises providing to the intercellular compartment surrounding a FAP cell an effective amount of a TGFβRII-Fc.

Any effective amount that inhibits differentiation into an adipocyte may be used, for example, 250 pM to 2.5 mM, 100 pm to 2.0 mM, or 50 pm to 1.0 mM. In some embodiments, the effective amount is about 250 pM, about 500 pM, about 2.5 nM, about 5 nM, about 25 nM, about 50 nM, about 250 nM, about 500 nM, about 2.5 µM, about 5 µM, about 25 µM, about 50 µM, about 250 µM, about 500 µM, and about 2.5 mM.

Further, the method can be performed *in vitro.* Additionally or alternatively, the method can be performed *in vivo.* These in vitro and in vivo aspects for inhibiting FAP differentiation are not according to the claimed invention.

In an embodiment, it has now been discovered that TGFβRII-Fc, can be used to achieve a variety of desirable outcomes, and to prevent, reverse, reduce the occurrence of, and/or treat FD in skeletal muscle.

FGFβRII-Fc may be administered or otherwise provided in a composition, such as a pharmaceutical composition further comprising one or more pharmaceutically acceptable excipients as described herein.

In further embodiments, the therapeutically effective amount of the TGFβRII-Fc is an amount that prevents, reverses, reduces the occurrence of, or treats FD.

The therapeutically effective amount of FGFβRII-Fc, or a composition comprising FGFβRII-Fc, may be administered one or more times depending on the identity and severity of the injury, surgery or condition.

FGFβRII-Fc may be administered after surgery.

The surgery may be a repair surgery, such as a tendon, ligament and/or muscle repair surgery. For example, the repair surgery may be RC surgery, Achilles tendon surgery. In a particular embodiment, the surgery is RC repair surgery.

Additionally or alternatively, TGFβRII-Fc or a composition comprising FGFβRII-Fc may be administered to a subject suffering from or at risk of suffering from age-related sarcopenia, obesity, metabolic syndrome, diabetes, and/or a neuropathy, such as peripheral neuropathy.

Additionally or alternatively, TGFβRII-Fc or a composition comprising FGFβRII-Fc may also be administered to a subject suffering from or at risk of suffering from a neurodegenerative disease, such as amyotrophic lateral sclerosis; a neuromuscular disease, such as a muscular dystrophy and inclusion body myositis; or chronic obstructive pulmonary disease; or the subject may be a chronic non-ambulatory stroke patient.

### EXAMPLES

The following examples are provided to further illustrate the invention disclosed herein but, of course, should not be construed as in any way limiting its scope. In each example the TGFβRII-Fc used was the short form of TGFβR fused to human IgG1 (see SEQ ID NO:3).

### Example 1 - in vivo mechanism of action; inhibition of adipogenic differentiation

Mechanistically, muscle infiltrating adipocytes are generated from muscle-resident fibro-adipogenic progenitor cells (FAPs) that are activated as part of the normal wound healing response. It was found that TGFβRII-Fc inhibits adipocyte differentiation from a FAPs-like cell-line, *i.e.,* 3T3-L1 cells (see Figs. 3A-3H). This supports the idea that TGFβRII-Fc works by preventing FAPs differentiation into adipocytes *in vivo.*

Cryopreserved murine 3T3-L1 pre-adipocytes, purchased from ZenBio, were thawed and seeded at approximately 10,000 cells/cm² in Preadipocyte Medium (PM: DMEM, high glucose, HEPES pH 7.4, 10 % Bovine Calf Serum (BCS), and Penicillin + Streptomycin (PS)). Cells were maintained at 37°C in a humidified incubator with 5% CO₂ until reaching 100% confluence, which takes in about four days. During this time, growth media was replaced every other day. Two days after reaching confluence, Preadipocyte Medium (PM) was replaced with an appropriate volume of Differentiation Medium (DM: DMEM, high glucose, sodium pyruvate, HEPES pH 7.4, 10 % Fetal Bovine Serum (FBS), 33 µM Biotin, 10 µg/ml Human insulin, 1 µM Dexamethasone, 0.5 mM 3-Isobutyl-1-methylxanthine (IBMX)) and incubated for 3 days. Differentiation Medium was then replaced with Adipocyte Maintenance Medium (MM: DMEM high glucose, sodium pyruvate, HEPES pH 7.4, 10 % FBS, 33 µM Biotin, 10 µg/ml Human insulin). Cells were maintained up to 10 days post-differentiation with medium exchange every other day.

In this assay 3T3-L1 cells were treated with TGFβRII-Fc beginning at different stages of differentiation. In one assay, confluent 3T3-L1cells were grown in PM, differentiated 3 days in DM and maintained up to 5 days in MM. 300 nM of TGFβRII-Fc was added at day 0 (beginning of DM treatment), or at day 3 (end of DM treatment), or at day 5 (after 2 days in MM). Cells were kept under treatment until the end of the experiment at day 8 or day 10.

At the end of the experiment adipocyte differentiation was measured by immunofluorescence. In this experiment, 10,000 3T3-L1 cells/cm² were plated in a 96-well plate in preadipocyte media. At day 0, cells were treated with TGFβRII-Fc. Treatments were continued in the appropriate medium to allow adipocyte differentiation until day 10. At day 10, cells were washed twice with PBS and fixed with 10% formalin for 30 min at RT. Cells were then washed twice with PBS, followed by staining with 0.01% saponin, 1 µg/ml Nile Red and 1 µg/ml DAPI in PBS for 15 min at RT. After staining, cells were washed 3 times with PBS. Images were taken with an Olympus Fluoview FC1000 confocal laser scanning microscope.

Results from this experiment are shown in Figs 3A-3M. 3T3-L1 cells were treated with a TGFβRII-Fc or vehicle *(i.e.,* PBS) on day 0, and differentiated for 8 days (Fig. 3A). The number of new adipocytes was reduced by 95% (Figs. 3B-3G). In Fig. 3B green represents Nile Red staining of lipid droplets and purple represents DAPI staining of nuclei. For quantitative Nile Red and DAPI fluorescence measurements, fluorescence is measured before and after staining according to published protocols. In this experiment 3T3-L1 cells were treated in quadruplicates in a 96 well plate. Multiple images were taken from each well. Number and size of lipid droplets, the average lipid area, normalized Nile-red fluorescence and lipolysis activity were calculated using Image J software from two biological replicates (Figs. 3C-3M).

In such an adipocyte differentiation experiment the extent of adipocyte formation can also be determined by gene expression analysis. In the experiment resulting in the data obtained in Figs. 3C-3H, 3T3-L1 cells were treated with TGFβRII-Fc or vehicle on day 0 and differentiated for 8 days. Total RNA was isolated from 3T3-L1 cells at day 0, day 3 and day 8, and cDNA was synthesized from RNA by reverse transcriptase using published protocols. To quantify expression of a selection of adipocyte marker genes primer pairs amplifying specific regions in these genes were synthesized and cDNA was analyzed by a Lightcycler 480 (Roche). For each experiment primer standard curves were created using a dilution series, spanning 4 orders of magnitude, from genomic DNA isolated from 3T3-L1 cells according to published protocols. The highest concentration of genomic DNA yields qRT-PCR amplification at around cycle 20 for the majority of primer pairs. A Tm-curve is also performed as a quality control for each primer pair at the end of each qRT-PCR run to verify that only a single amplicon is produced by each primer pair and that no primer dimers are formed. Data are usually obtained from at least two biological replicates and four technical replicates.

### Example 2 - mouse diet-induced obesity (DIO) model

TGFβRII-Fc inhibits FI of skeletal muscles in a mouse DIO model, as shown in Figs. 4A-4J. In this model, mice are placed on a high fat diet (HFD) for several weeks. After 4 weeks on HFD mice show early signs of developing obesity. One of these signs is an increase of FI of skeletal muscles. FI is the first manifestation of FD in DIO, with muscle atrophy developing later. New fat cells are generated by adipogenic differentiation of fibro-adipogenic progenitor cells (FAPs) that reside in the muscle and can be detected by histopathologic and gene expression analyses.

In this experiment a total of 20 8-week old, male C57BL/6 mice, obtained from Charles River Laboratories, were used. Animals were housed under supervision of the Michigan State University (MSU) Campus Animal Resources and allowed to acclimate for seven days prior to the start of the study. Upon arrival all animals were provided with PMI Nutrition International Certified Rodent Chow No. 5CR4. At the beginning of the experiment 14 mice were placed on a high fat diet, (Research Diet D12492, 60% fat kcal). This diet was provided *ad libitum* throughout the remainder of the study. Regular chow (PMI Nutrition International Certified Rodent Chow No. 5CR4) was provided *ad libitum* to 6 control mice throughout the study. Mice on high fat diet were separated into two groups and 7 mice were treated with TGFβRII-Fc and 7 with vehicle *(i.e.,* PBS) for 4 weeks. Dosing with TGFβRII-Fc or vehicle control was done by bi-weekly subcutaneous injections. TGFβRII-Fc was provided as a stock of 3 mg/ml and dosed at 15 mg/kg body weight. TGFβRII-Fc aliquots were kept frozen (-80°C) until used. On the day of dosing, an aliquot was moved from -80°C to the bench at room temperature at least one hour before dosing. Thawed samples were gently mixed by inverting the tube to ensure homogeneous distribution followed by centrifugation to collect the liquid from the cap. Vortexing or harsh mixing methods were avoided to prevent protein denaturation or bubble formation. Vehicle control was given to appropriate animals at the same time as TGFβRII-Fc. The experiment was performed under supervision of the IACUC of MSU to ensure animal safety. At the end of the experiment mice were sacrificed by CO₂ asphyxiation. At necropsy leg muscles were extracted and flash frozen in liquid nitrogen.

FI of skeletal muscles can be determined in such an experiment by histopathologic analysis of muscles. For this analysis muscles can be frozen in liquid nitrogen and then fixed in paraformaldehyde followed by cryoprotection with sucrose. Cryosections of the muscles can then be stained for hematoxylin and Oil Red O (ORO). In this assay ORO detects intermuscular and intramuscular fat. Intermuscular fat is the result of FI, while intramuscular fat is a result of lipid accumulation within muscle fibers. In the DIO model accumulation of both intermuscular and intramuscular fat can be detected.

In this experiment, shown in Fig. 4A, quadriceps muscles of mice fed a HFD for 4 weeks and treated with TGFβRII-Fc or vehicle control were analyzed by histopathology. Quadriceps muscles were placed into a 15 ml tube with 8 ml of 4% paraformaldehyde in phosphate buffer solution (no saline) and kept for 48 h at RT. Muscles were then transferred to fresh 15 ml tubes with 8 ml of 30% sucrose in phosphate buffer solution (no saline) and kept at 4°C until the muscles sank to the bottom of the tube *(i.e.,* 4 days). Fixed and sucrose cryoprotected muscles were then embedded in OCT and cryosectioned at 12 µm at -20°C. Sections were stained with ORO and counterstained with hematoxylin. Quantification of ORO was done using Image J on images obtained with an OlyVIA system at 0.7x magnification. Inhibition of FI by a TGFβRII-Fc under these conditions was evidenced by a 70-80% reduction in total ORO staining as well as a 65-70% reduction in intermuscular ORO (Fig. 4A-4D).

In this type of an experiment, FI of skeletal muscles can also be determined by gene expression analysis. RNA was isolated from a portion of the belly of the appropriate muscle (20-25 mg) using the ReliaPrep^{™} RNA tissue miniprep system from Promega and a tissue homogenizer. Tissue was placed into a 2 ml screw cap tube and two beads and 1.3 ml TRIzol^{™} Reagent was added. Tissue is homogenized 2 x for 4 minutes using a mixer mill (Retsch) at a frequency of 25 s⁻¹. Samples were placed on ice in between cycles. 260 µl chloroform was added and the samples shaken vigorously by hand until the solution was a cloudy pink color. After centrifugation at 4°C for 10' at 15k x g an aliquot of 750 µl of the aqueous phase was transferred to a new tube, avoiding the interphase. 340 µl of ice-cold isopropanol was added, and the sample was mixed by vortexing. The sample was then transferred onto a Promega RNA Cell Miniprep column and processed according to the manufacturer's instructions. A DNAse 1 treatment step was performed on the column for 30 min. RNA was eluted in 15 µl RNase-free H₂O. The RNA concentration and the purity of each sample were determined by Nanodrop. For cDNA synthesis 1µg of total isolated RNA was used from each sample and reverse transcribed using the Multiscribe^{™} Reverse Transcriptase enzyme kit (from ThermoFischer). cDNA was then analyzed as previously described using primers for adipocyte markers on a Roche Lightcycler^{®} 480. Data was then normalized to a household gene (*Rpl4*)*.*

In this experiment gene expression was analyzed in the quadriceps muscles of mice that had been on a HFD for 4 weeks and treated with TGFβRII-Fc or vehicle control. Treatment with TGFβRII-Fc significantly inhibited FI under these conditions, as evidenced by 5 to 60-fold reduction in expression of the adipocyte marker genes *Adipoq, Cidec, Fabp4, Lep, Plin1* and *Pparg* (Fig. 4E-4J).

Surprisingly, it was found that inhibition of both TGF-β1 and β3 prevents adipogenesis, as shown in Figs. 3A-3H and Figs. 4A-4J, a counterintuitive result considering previous reports that TGF-β1 itself inhibits adipogenesis by Ignotz, R.A. and Massague, J. Proc Natl Acad Sci U S A. 1985 Dec;82(24):8530-4. doi: 10.1073/pnas.82.24.8530.). Further, because TGFβRII-Fc does not inhibit TGF-β2 signaling, the administration of TGFβRII-Fc before, during and/or after, for example, RC repair surgery may inhibit, prevent or reduce the occurrence of FD of muscles without interfering with tendon regeneration. Thus, TGFβRII-Fc may also facilitate tendon regeneration and attachment while inhibiting FD. TGFβRII-Fc can also be used to halt FD of muscles in patients, where RC repair surgery is not recommended and conservative treatment is indicated.

### Example 3 - Inhibition of fatty degeneration of skeletal muscles in a mouse model of rotator cuff injury

In this experiment the tendons of two RC muscles, the supraspinatus and the infraspinatus muscles, are resected to approximate the RC tendon tears found in human patients. This mouse model of RC injury is a good mimic for RC injuries in humans, since mice also develop significant FD including FI and muscle atrophy in the shoulder muscles after 6 weeks, as well as fibrosis/scarring.

In this experiment surgery to induce RC injury was done by RC tendon transection and denervation. Surgical procedures were performed under general anesthesia with 2.5% isoflurane and oxygen. The surgical interventions in the RC were performed in the right shoulder of the mice, and a sham surgery was performed in the left shoulder. For surgery mice were placed on a surgical table, and the clavicle bone and the deltoid muscle were exposed through a skin incision. The trapezius muscle was separated to expose the suprascapular nerve, and a segment of the nerve 5 mm long was resected to prevent the reattachment and healing of the nerve. The deltoid muscle was longitudinally split to expose the rotator cuff tendons at the shoulder joint. The supraspinatus and infraspinatus tendons were completely transected with removal of the tendon portion to decrease the chance of incidental healing. The deltoid and trapezius muscles and skin incision were closed. For the sham procedure on the contralateral shoulder, the skin and muscle incisions were performed, the rotator cuff and/or suprascapular nerve identified, and the muscle and skin closed again. Surgery typically last approximately 20 min per mouse.

In this experiment surgery to induce RC injury was performed on 24 female 12-week old C57BL/6 mice obtained from Jackson Laboratories. Starting two days after surgery 12 mice were treated with TGFβRII-Fc, and 12 mice were treated with vehicle control *(i.e.,* PBS) for 6 weeks. The study was performed at the MSU In Vivo Facility under supervision of MSU's IACUC to ensure animal safety.

At the beginning of the experiment animals were housed under supervision of the MSU Campus Animal Resources. Animals were allowed to acclimate for seven days prior to the start of the study. Upon arrival, PMI Nutrition International Certified Rodent Chow No. 5CR4 was provided and given to the animals *ad libitum* for the duration of the study. On the day before the study the animals were weighed and randomized into two treatment groups in such a way as to generate cohorts with no significant difference between them with respect to their body weights. Animals were singly housed for the study after cohorts were generated. Mortality/moribundity checks were performed twice daily, once in the morning and once in the evening, for two days after surgery and once daily for all subsequent days of the study. Post-operative care included cleansing of the surgery incisions with chlorhexidine solution daily for two days after surgery. Animals were assessed daily post operatively for use of limb and weighed daily to assess feed intake. For post-operative pain management 1 mg/kg buprenorphine was injected subcutaneously every 12 hours for two days following surgery.

In this experiment TGFβRII-Fc or vehicle was administered bi-weekly to animals starting two days after surgery for 6 weeks. TGFβRII-Fc or vehicle was administered by subcutaneous route. TGFβRII-Fc was provided as a stock of 3 mg/mL and dosed at 15 mg/kg body weight. TGFβRII-Fc aliquots were kept frozen (-80°C) until used. On the day of dosing, an aliquot was moved from -80°C to the bench at room temperature at least one hour before dosing. Thawed samples were gently mixed by inverting the tube to ensure homogeneous distribution followed by centrifugation to collect the liquid from the cap. Vortexing or harsh mixing methods were avoided to prevent protein denaturation or bubble formation. Vehicle control *(i.e.,* PBS) was given to appropriate animals at the same time as TGFβRII-Fc treatment.

At the end of the experiment animals were sacrificed by CO₂ asphyxiation. At necropsy the shoulder girdle and upper limbs were excised *en block* with the muscles and connective tissues. The supraspinatus and infraspinatus muscles from the injured and sham operated sides were separated from bones and connective tissues and immediately weighed. Wet weights were recorded and the weight of the operated muscle was calculated as a percentage of the sham operated control muscle. Statistical significance of differences between muscle weights from TGFβRII-Fc and vehicle treated animals was determined by a Student's t-test (n=6).

In this experiment treatment TGFβRII-Fc significantly reduced atrophy of the supraspinatus (SS) and infraspinatus (IS) muscles after RC injury (see Table 1 and Fig. 5A). Vehicle treated animals lost on average about 30% of their SS muscle weight and about 55% of their IS muscle weight. In contrast, muscles from animals treated with TGFβRII-Fc lost only around 10% and 13% of their SS and IS muscle weight respectively. This corresponds to a 70-80% reduction of muscle atrophy in mice treated with TGFβRII-Fc compared to control mice.

**Table 1. Shoulder Muscle Weights at Tissue Harvest**

| **Group No.** | **Animal No.** | **muscle** | **left side (g)^{a}** | **right side (g)^{b}** | **% loss of muscle weight^{C}** |
|---|---|---|---|---|---|
| 1 | 1004 | supraspinatus | 0.0414 | 0.0337 | 18.6 |
| 1 | 1005 | supraspinatus | 0.0288 | 0.0212 | 26.4 |
| 1 | 1006 | supraspinatus | 0.0389 | 0.0213 | 45.2 |
| 1 | 1010 | supraspinatus | 0.0257 | 0.0153 | 40.5 |
| 1 | 1011 | supraspinatus | 0.0268 | 0.0173 | 35.4 |
| 1 | 1012 | supraspinatus | 0.0327 | 0.0242 | 26.0 |
| 2 | 2004 | supraspinatus | 0.0296 | 0.0237 | 19.9 |
| 2 | 2005 | supraspinatus | 0.0310 | 0.0233 | 24.8 |
| 2 | 2006 | supraspinatus | 0.0242 | 0.0288 | -19.0^{d} |
| 2 | 2010 | supraspinatus | 0.0312 | 0.0313 | -0.3 ^{d} |
| 2 | 2011 | supraspinatus | 0.0346 | 0.0333 | 3.8 |
| 2 | 2012 | supraspinatus | 0.0388 | 0.0289 | 25.5 |
| 1 | 1004 | infraspinatus | 0.0433 | 0.0176 | 59.4 |
| 1 | 1005 | infraspinatus | 0.0265 | 0.0136 | 48.7 |
| 1 | 1006 | infraspinatus | 0.0303 | 0.0144 | 52.5 |
| 1 | 1010 | infraspinatus | 0.0351 | 0.0127 | 63.8 |
| 1 | 1011 | infraspinatus | 0.0319 | 0.0168 | 47.3 |
| 1 | 1012 | infraspinatus | 0.0218 | 0.0102 | 53.2 |
| 2 | 2004 | infraspinatus | 0.0231 | 0.0158 | 31.6 |
| 2 | 2005 | infraspinatus | 0.0198 | 0.0178 | 10.1 |
| 2 | 2006 | infraspinatus | 0.0219 | 0.0159 | 27.4 |
| 2 | 2010 | infraspinatus | 0.0270 | 0.0325 | -20.4 ^{d} |
| 2 | 2011 | infraspinatus | 0.0303 | 0.0293 | 3.3 |
| 2 | 2012 | infraspinatus | 0.0292 | 0.0213 | 27.1 |

| | | | | | |
|---|---|---|---|---|---|
| ^{a} Sham surgery ^{b} Induced RC Injury surgery ^{c} % loss of right side compared to left side ^{d} negative number means a gain in weight in right side compared to left side | | | | | |

Reduction of FI in shoulder muscles after RC injury by TGFβRII-Fc, can also be shown using adipocyte marker gene expression analysis. In this experiment the SS and IS muscles from the injured and sham operated sides were flash frozen in liquid nitrogen. RNA was isolated from an entire muscle using the ReliaPrep^{™} RNA tissue miniprep system from Promega and a tissue homogenizer, followed by cDNA synthesis as described. Treatment with TGFβRII-Fc reduced FI in the SS and infraspinatus IS muscles, as shown by 2-3 fold reduction in *Cidec* and *Lep* gene expression (Fig. 5B and 5C). Significance was determined by a Student's t-test (n=4).

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. TGFβRII-Fc for use in the prevention or treatment of fatty degeneration of skeletal muscle in a subject in need thereof.

2. TGFβRII-Fc for use according to claim 1, wherein the TGFβRII-Fc inhibits fatty degeneration while also facilitating tendon regeneration.

3. TGFβRII-Fc for use according to claim 1, wherein the subject has or is at risk of having age-related sarcopenia; obesity; metabolic syndrome; diabetes; and/or a peripheral neuropathy.

4. TGFβRII-Fc for use according to claim 1, wherein the subject has a neurodegenerative disease, such as amyotrophic lateral sclerosis; a neuromuscular disease, such as a muscular dystrophy and inclusion body myositis; or chronic obstructive pulmonary disease.

5. TGFβRII-Fc for use according to claim 1, wherein the subject is a chronic non-ambulatory stroke patient.

## Patentansprüche

1. TGFβRII-Fc zur Verwendung bei der Prävention oder Behandlung von fettiger Degeneration von Skelettmuskel bei einem Subjekt, das dessen bedarf.

2. TGFβRII-Fc zur Verwendung nach Anspruch 1, wobei das TGFβRII-Fc fettige Degeneration hemmt und gleichzeitig die Sehnenregeneration fördert.

3. TGFβRII-Fc zur Verwendung nach Anspruch 1, wobei das Subjekt altersbedingte Sarkopenie; Fettleibigkeit; metabolisches Syndrom; Diabetes; und/oder eine periphere Neuropathie hat oder bei dem ein Risiko dafür besteht.

4. TGFβRII-Fc zur Verwendung nach Anspruch 1, wobei das Subjekt eine neurodegenerative Erkrankung, wie beispielsweise amyotrophe Lateralsklerose; eine neuromuskuläre Erkrankung, wie beispielsweise eine Muskeldystrophie und Einschlusskörpermyositis; oder eine chronisch obstruktive Lungenerkrankung hat.

5. TGFβRII-Fc zur Verwendung nach Anspruch 1, wobei das Subjekt ein chronischer, mobil eingeschränkter Schlaganfallpatient ist.

## Revendications

1. TGFβRII-Fc pour une utilisation dans la prévention ou le traitement de la dégénérescence graisseuse du muscle squelettique chez un sujet en ayant besoin.

2. TGFβRII-Fc pour une utilisation selon la revendication 1, dans laquelle TGFβRII-Fc inhibe la dégénérescence graisseuse tout en facilitant également la régénération tendineuse.

3. TGFβRII-Fc pour une utilisation selon la revendication 1, dans laquelle le sujet présente ou est à risque de présenter une sarcopénie liée à l'âge ; une obésité ; un syndrome métabolique ; un diabète ; et/ou une neuropathie périphérique.

4. TGFβRII-Fc pour une utilisation selon la revendication 1, dans laquelle le sujet présente une maladie neurodégénérative, telle que la sclérose latérale amyotrophique ; une maladie neuromusculaire, telle qu'une dystrophie musculaire et une myosite à inclusions ; ou une bronchopneumopathie chronique obstructive.

5. TGFβRII-Fc pour une utilisation selon la revendication 1, dans laquelle le sujet est un patient chronique non ambulatoire ayant subi un accident vasculaire cérébral.
